# EUROPEAN PATENT APPLICATION

(11) **EP 2 626 347 A1**
(43) Date of publication of application: **14.08.2013**
(21) Application number: 11830239.7
(22) Date of filing: 08.10.2011
(51) Int. Cl.: C07D 213/73, A61K 31/44, A61P 25/00

(54) **DALFAMPRIDINE POLYMORPH, PREPARATION THEREFOR, AND APPLICATION THEREOF**

(30) Priority: 08.10.2010 CN 201010298651
(71) Applicant: Tianjin Hemay Bio-Tech Co., Ltd., Tianjin 300457 (CN); Tianjin Michele Sci-Tech Development Co., Ltd., Tianjin 300192 (CN)
(72) Inventor: ZHANG, Hesheng, Tianjin 300457 (CN); CHEN, Xin, Tianjin 300457 (CN)
(74) Representative: Brand, Thomas Louis
(86) International application number: PCT/CN2011/080548
(87) International publication number: WO 2012/045283

(57) **Abstract**

Provided are polymorphs I to VIII of a dalfampridine or of its solvates, a preparation method therefore, and applications thereof as an active component of a medicine. Said polymorphs I to VIII of the dalfampridine or of its solvents can be used to prepare formulations for improving the walking speed of patients of multiple sclerosis and for alleviating other symptoms of multiple sclerosis.

## Description

### FIELD OF THE INVENTION

The present invention relates to a field of pharmaceutical technology, and in particular relates to polymorphs of 4-aminopyridine or polymorphs of solvates thereof, methods for preparing the same, and uses thereof.

### BACKGROUND OF THE INVENTION

Multiple sclerosis is a chronic, often disabling autoimmune disease. According to the statistic data from the Multiple Sclerosis International Federation, more than 2.5 million people worldwide have been diagnosed with the disease, and as many as 400,000 people in the United States suffer from the disease. The clinic symptoms of multiple sclerosis mainly include progressive difficulty in walking, fatigue, lack of physical coordination, impaired eyesight and memory deterioration, and heat sensitivity. Relapsing remitting form is the most common one in all forms of multiple sclerosis, which is mainly characterized by unpredictable acute attacks followed by periods of months to years of remission, with no new signs of disease. The secondary form of multiple sclerosis is progressive form. From the onset of multiple sclerosis, a patient's condition becomes progressively serious with no periods of remission. Progressive form of multiple sclerosis is mainly characterized by a permanent and irreversible neurodegenerative progress. However, no matter what forms of multiple sclerosis a patient has, it is an important symptom that the patient's walking speed becomes slow in the progress of multiple sclerosis. The patient's slow walking speed limits his autonomic activity and affects his normal daily life.

At present, drugs for treating multiple sclerosis work mainly by decreasing inflammatory impairment in the central nervous system, most of which are administered parenterally.

Recently, Ampyra(4-aminopyridine, dalfampridine), a novel drug for treating multiple sclerosis by oral administration, was approved by the FDA and marketed in the United States. Ampyra was the first drug for treating multiple sclerosis that can be taken orally, and was also the first FDA-approved drug to improve walking speed in multiple sclerosis patients.

Research on Ampyra dates back to the 1960s, when Dr. Floyd Davis found from an unusual clinical observation that many multiple sclerosis patients fare better when their body temperatures were slightly lowered. In many multiple sclerosis patients, the myelin sheath that wraps around nerve fibers in the brain and spinal cord is damaged, essentially causing a short circuit. However, lower body temperature enabled electrical pulses to continue its travel along nerve fibers. This finding is very important because it shows that the damaged nerve fibers were not doomed, as previously believed.

The research team subsequently launched a series of laboratory studies to understand the mechanism that explained this phenomenon, then tried to look for a compound that could mimic some of the effect of lower body temperature on nerve fibers, and learned of some drugs which could block the potassium ion channels in nerve fibers, such as 4-aminopyridine(dalfampridine).

In 1983, dalfampridine was injected in 11 patients whose eyesight and motor function were impaired because of multiple sclerosis, and the therapeutic efficiency was stunning. The patients' walking speed and eyesight both were improved.

In Phase III clinical trials, the drug yielded a consistent improvement in walking speed. The results of a series of clinical trials showed that the walking speed in multiple sclerosis patients treated with dalfampridine was faster than that in patients who took a placebo, and that in patients who took a prescribed dosage of dalfampridine, the number of patients whose walking speed was increased by at least 10% to 30% compared with the baseline level was obviously higher than that of those who took a placebo.

It was also found in the researches that, although dalfampridine has been approved specifically for the treatment of impaired walking in a multiple sclerosis patient, it also relieves other symptoms of multiple sclerosis, since it restores signal conduction in all the affected nerve fibers.

It is well known that a crystalline form of a compound has a better stability than an amorphous form thereof, which can extend a shelf life of a drug substance, and is more suitable for preparing a formulation. As a result, a crystalline form is a best choice of a physical form of a drug substance, particularly for air- and moisture-sensitive compound, such as 4-aminopyridine. However, no crystalline form of 4-aminopyridine, for example a polymorph thereof, is reported up to now.

### SUMMARY OF THE INVENTION

One object of the present invention is to provide polymorphs of 4-aminopyridine or polymorphs of solvates thereof.

Another object of the present invention is to provide methods for preparing polymorphs of 4-aminopyridine or polymorphs of solvates thereof and uses thereof.

In order to achieve the above objects, the technical solutions of the present invention are provided as follows.

The polymorphs of 4-aminopyridine or the polymorphs of the solvates thereof according to the present invention can be prepared by one of the following methods.
1. 4-Aminopyridine was dissolved in a single solvent by heating, and the resulting clear solution was stood and cooled to precipitate a solid.
2. 4-Aminopyridine was dissolved in a mixed solvent by heating, and the resulting clear solution was stood and cooled to precipitate a solid.
3. 4-Aminopyridine was dissolved in a single solvent by heating, and the resulting clear solution was cooled under stirring to precipitate a solid.
4. 4-aminopyridine was dissolved in a single solvent by heating, and then a poor solvent was added dropwise to the resulting clear solution under stirring and cooling to precipitate a solid.
5. 4-Aminopyridine was dissolved in a mixed solvent by heating, and the resulting clear solution was cooled under stirring to precipitate a solid.
6. 4-aminopyridine was dissolved in a mixed solvent by heating, and then the resulting clear solution was cooled under stirring, and meanwhile a poor solvent is added dropwise thereto to precipitate a solid.

In the above preparation methods, if no solid was precipitated when the solution is cooled to room temperature, the solution can be cooled to a temperature in the range of from 0 °C to 10 °C in a refrigerator.

The term "single solvent" used herein refers to any solvent selected from the group consisting of water, alcohols, esters, ketones, ethers, alkanes, halogenated alkanes, nitriles, aromatic hydrocarbons, amides, sulphones, cyclic ethers, pyridines and sulphoxides, including but not limited to any one of methanol, ethanol, propanol, isopropanol, ethylene glycol, propylene glycol, acetone, butanone, diethyl ether, isopropyl ether, tetrahydrofuran, 1,4-dioxane, ethyl formate, ethyl acetate, propyl formate, isopropyl formate, methyl acetate, propyl acetate, isopropyl acetate, butyl formate, N,N-dimethylformamide, N,N-dimethylacetamide, dimethyl sulphoxide, water, pyridine, acetonitrile, benzene, toluene, xylol, ethylene glycol dimethyl ether, ethylene glycol monomethyl ether, ethylene glycol diethyl ether, ethylene glycol monoethyl ether, ethylene glycol dipropyl ether, ethylene glycol monopropyl ether, n-hexane, chloroform, dichloromethane, 1,2-dichloroethane and carbon tetrachloride.

The term "mixed solvent" used herein refers to a mixed solution of two or more solvents selected from the group consisting of water, alcohols, esters, ketones, ethers, alkanes, halogenated alkanes, nitriles, aromatic hydrocarbons, amides, sulphones, cyclic ethers, pyridines and sulphoxides, specifically including but not limited to a mixed solution of two or more solvents selected from the group consisting of methanol, ethanol, propanol, isopropanol, ethylene glycol, propylene glycol, acetone, butanone, diethyl ether, isopropyl ether, tetrahydrofuran, 1,4-dioxane, ethyl formate, ethyl acetate, propyl formate, isopropyl formate, methyl acetate, propyl acetate, isopropyl acetate, butyl formate, N,N-dimethylformamide, N,N-dimethylacetamide, dimethyl sulphoxide, water, pyridine, acetonitrile, benzene, toluene, xylol, ethylene glycol dimethyl ether, ethylene glycol monomethyl ether, ethylene glycol diethyl ether, ethylene glycol monoethyl ether, ethylene glycol dipropyl ether, ethylene glycol monopropyl ether, n-hexane, chloroform, dichloromethane, carbon tetrachloride and petroleum ether. If the mixed solvent is a mixture of above-mentioned two solvents, a ratio of the two solvents is in the range of from 100:1 to 1:100.

The term "poor solvent" used herein refers to one or more solvents selected from the group consisting of water, alcohols, esters, ketones, ethers, alkanes, halogenated alkanes, nitriles, aromatic hydrocarbons, amides, sulphones, cyclic ethers and sulphoxides, including but not limited to one or more solvents selected from the group consisting of methanol, ethanol, propanol, isopropanol, ethylene glycol, propylene glycol, acetone, butanone, diethyl ether, isopropyl ether, tetrahydrofuran, 1,4-dioxane, ethyl formate, ethyl acetate, propyl formate, isopropyl formate, methyl acetate, propyl acetate, isopropyl acetate, butyl formate, water, pyridine, acetonitrile, benzene, toluene, xylol, ethylene glycol dimethyl ether, ethylene glycol monomethyl ether, ethylene glycol diethyl ether, ethylene glycol monoethyl ether, ethylene glycol dipropyl ether, ethylene glycol monopropyl ether, n-hexane, chloroform, dichloromethane, carbon tetrachloride and petroleum ether. If the poor solvent is a mixture of above-mentioned two solvents, a ratio of the two solvents is in the range of from 100:1 1 to 1:100.

The present invention discloses eight polymorphs of 4-aminopyridine or of the solvates thereof, i.e. polymorphs I to VIII.

In the present application, X-ray Powder Diffraction is performed under the following measurement conditions

| | |
|---|---|
| Sample Weight: | about 100 mg |
| Target: | Cu |
| Filter Disk: | monochromatic filter |
| Voltage/Current: | 40kV/100mA |
| Slit: | SS/DS 1°, RS 0.3 mm |
| Scanning Rate: | 8 °/min |
| Range: | 3-50 |

Peaks with peak intensity equal or greater than 15 in X-ray powder diffraction patterns of the eight polymorphs of 4-aminopyridine or of the solvates thereof according to the present invention are shown in the following tables.
(1) Polymorph I of 4-aminopyridine or of the solvates thereof
Peaks in X-ray powder diffraction pattern thereof have the following features:

| Diffraction Angle (2θ, °) | Intensity (I/I₀) | Diffraction Angle (2θ, °) | Intensity (I/I₀) |
|---|---|---|---|
| 18.940 | 83.0 | 20.040 | 26.7 |
| 24.381 | 100.0 | 29.601 | 32.1 |

(2) Polymorph II of 4-aminopyridine or of the solvates thereof
Peaks in X-ray powder diffraction pattern thereof have the following features:

| Diffraction angle (2θ, °) | Intensity (I/I₀) | Diffraction angle (2θ, °) | Intensity (I/I₀) |
|---|---|---|---|
| 20.101 | 88.1 | 21.380 | 15.9 |
| 24.361 | 100 | 29.260 | 16.3 |
| 30.140 | 31.9 | 33.039 | 27.1 |

(3) Polymorph III of 4-aminopyridine or of the solvates thereof
Peaks in X-ray powder diffraction pattern thereof have the following features:

| Diffraction angle (2θ, °) | Intensity (I/I₀) | Diffraction angle (2θ, °) | Intensity (I/I₀) |
|---|---|---|---|
| 18.980 | 100.0 | 24.321 | 21.7 |
| 38.619 | 17.5 | | |

(4) Polymorph IV of 4-aminopyridine or of the solvates thereof
Peaks in X-ray powder diffraction pattern thereof have the following features:

| Diffraction angle (2θ, °) | Intensity (I/I₀) | Diffraction angle (2θ, °) | Intensity (I/I₀) |
|---|---|---|---|
| 20.020 | 100.0 | 20.119 | 99.3 |
| 24.259 | 93.4 | 24.419 | 98.5 |
| 29.200 | 26.7 | | |

(5) Polymorph V of 4-aminopyridine or of the solvates thereof
Peaks in X-ray powder diffraction pattern thereof have the following features:

| Diffraction angle (2θ, °) | Intensity (I/I₀) | Diffraction angle (2θ, °) | Intensity (I/I₀) |
|---|---|---|---|
| 14.160 | 35.4 | 18.979 | 33.0 |
| 19.980 | 48.8 | 21.340 | 34.5 |
| 24.361 | 100.0 | 29.259 | 19.6 |

(6) Polymorph VI of 4-aminopyridine or of the solvates thereof
Peaks in X-ray powder diffraction pattern thereof have the following features:

| Diffraction angle (2θ, °) | Intensity (I/I₀) | Diffraction angle (2θ, °) | Intensity (I/I₀) |
|---|---|---|---|
| 20.179 | 76.5 | 24.440 | 100.0 |
| 29.300 | 30.0 | | |

(7) Polymorph VII of 4-aminopyridine or of the solvates thereof
Peaks in X-ray powder diffraction pattern thereof have the following features:

| Diffraction angle (2θ, °) | Intensity (I/I₀) | Diffraction angle (2θ, °) | Intensity (I/I₀) |
|---|---|---|---|
| 20.019 | 100.0 | 24.319 | 46.6 |
| 29.120 | 27.4 | | |

(8) Polymorph VIII of 4-aminopyridine or of the solvates thereof
Peaks in X-ray powder diffraction pattern thereof have the following features:

| Diffraction angle (2θ, °) | Intensity (I/I₀) | Diffraction angle (2θ, °) | Intensity (I/I₀) |
|---|---|---|---|
| 20.060 | 50.5 | 24.401 | 60.6 |
| 29.280 | 100.0 | | |

The polymorphs of 4-aminopyridine or the polymorphs of the solvates thereof according to the present invention may be in a form of an individual polymorph or a mixture of two or more polymorphs when in use.

A unit dosage of the polymorphs of 4-aminopyridine or the polymorphs of the solvates thereof according to the present invention when used as an active ingredient is in the range of from 0.01mg to 500 mg, preferably from 0.1 mg to 100 mg.

The term "unit dosage" as used herein refers to a unit which can be administered to a patient and easily operated and packed, i.e. a single dosage.

The polymorphs of 4-aminopyridine or the polymorphs of the solvates thereof according to the present invention when used as an active ingredient can treat multiple sclerosis, including but not limited to mild multiple sclerosis, relapsing-remitting multiple sclerosis, relapsing-progressive multiple sclerosis and chronic progressive multiple sclerosis.

The polymorphs of 4-aminopyridine or the polymorphs of the solvates thereof according to the present invention when used as an active ingredient can be formulated into various dosage forms with an appropriate pharmaceutical excipient. The dosage forms include but not limited to tablets (including but not limited to conventional tablets, buccal tablets, effervescent tablets, enteric-coated tablets, sustained release tablets, controlled release tablets, and orally disintegrating tablets), capsules (including but not limited to hard capsules and soft capsules), powders for injection (including but not limited to conventional powders for injection and lyophilized powders for injection), solutions, aerosols, sprays, creams, patches, eye drops, ear drops, granules, lyophilized rapid-dissolving tablets, implants, suppositories, and the like, which are suitable for oral, injection, inhalation, eye drop, ear drop, transdermal, rectal, vaginal administration and the like.

The appropriate pharmaceutical excipient according to the present invention includes but not limited to a disintegrating agent, a filling agent, a colorant, a flavoring agent, a lubricant, a glidant, an adhesive agent, an effervescing agent, a preservative agent, a solvent and/or a cosolvent.

The disintegrating agent used herein includes but not limited to any one or more of starch, low-substituted hydroxypropyl cellulose (L-HPC), crosslinked polyvinylpyrrolidone (PVPP), croscarmellose sodium (CCNa), crosslinked sodium carboxymethyl starch (CCMS-Na) and treated agar (TAG).

The filling agent used herein includes but not limited to any one or more of gelatin, sodium chloride, dextranum, glucose, maltose, sucrose, sorbitol, hydroxyethyl cellulose, dextrin, microcrystalline cellulose (MCC), lactose, mannitol, erythritol, starch and icing sugar.

The colorant used herein includes but not limited to any one or more of carotene, sunset yellow, lemon yellow, carmine and chlorophyll.

The flavoring agent used herein includes but not limited to any one or more of a natural flavoring agent and an artificial flavoring agent, such as stevioside, xylito, aspartame, sweet orange solid essence, sodium cyclamate, fruit essence, sucrose, glucose, menthol, citric acid, vitamin C and sorbose.

The lubricant used herein includes but not limited to any one or more of magnesium stearate, talcum powder, calcium stearate, stearic acid, polyethylene glycol 4000, polyethylene glycol 6000, hydrogenated vegetable oil, polyoxyethylene monostearate, light mineral oil, waxes, glycerol triacetate and magnesium dodecyl sulfate.

The glidant used herein includes but not limited to any one or more of micropowder silica gel, Cab-O-sil (Cabot Company), Arosil (Degussa Company) and hydrated sodium silicoaluminate.

The adhesive agent used herein includes but not limited to any one or more of water, starch, hydroxypropyl methyl cellulose (HPMC), sodium carboxymethyl cellulose (CMC-Na), hydroxy propyl cellulose (HPC), methylcellulose (MC), ethylcellulose (EC), polyvinylpyrrolidone (PVP) and ethanol.

An acid source in the effervescing agent used herein includes but not limited to any one or more of citric acid, tartaric acid, tetraacefic acid, lysine and arginine.

A base source in the effervescing agent used herein includes but not limited to any one or more of sodium bicarbonate, sodium carbonate, potassium bicarbonate and potassium carbonate.

The preservative agent used herein includes but not limited to any one or more of nipagins, benzoic acid, sodium benzoate and sorbic acid, wherein the nipagins include but not limited to any one or more of methylparaben, ethylparaben, propylparaben and butylparaben.

The solvent used herein includes but not limited to any one or more of water, polyethylene glycol 200, polyethylene glycol 300, polyethylene glycol 400, polyethylene glycol 600 and glycerol.

The cosolvent used herein includes but not limited to any one or more of poloxamer, ethanol, 1,2-propylene glycol, glycerol, polyethylene glycol 200, polyethylene glycol 300, polyethylene glycol 400 and polyethylene glycol 600.

The polymorphs of 4-aminopyridine or the polymorphs of the solvates thereof according to the present invention were exposed to the air at room temperature for 12 months, and then purities thereof were measured by using high performance liquid chromatography. A result shows that no degradation impurity was produced, which suggests that the polymorphs according to the present invention have highly excellent stability and are preferable physical forms of the drug substance.

### BRIEF DESCRIPTIONS OF THE DRAWINGS

Fig.1 shows X-ray powder diffraction pattern of the polymorph I of 4-aminopyridine or of the solvates thereof.

Fig.2 shows X-ray powder diffraction pattern of the polymorph II of 4-aminopyridine or of the solvates thereof.

Fig.3 shows X-ray powder diffraction pattern of the polymorph III of 4-aminopyridine or of the solvates thereof.

Fig.4 shows X-ray powder diffraction pattern of the polymorph IV of 4-aminopyridine or of the solvates thereof.

Fig.5 shows X-ray powder diffraction pattern of the polymorph V of 4-aminopyridine or of the solvates thereof.

Fig.6 shows X-ray powder diffraction pattern of the polymorph VI of 4-aminopyridine or of the solvates thereof.

Fig.7 shows X-ray powder diffraction pattern of the polymorph VII of 4-aminopyridine or of the solvates thereof.

Fig.8 shows X-ray powder diffraction pattern of the polymorph VIII of 4-aminopyridine or of the solvates thereof.

### DETAILED DESCRIPTION OF THE INVENTION

The melting point detector used herein is BUCHI-B-545;

The melting point tube used herein is 0.9-1.1mm melting point tube produced by Instrument Factory of West China University of Medical Sciences.

### Example 1: Preparation of Polymorph I

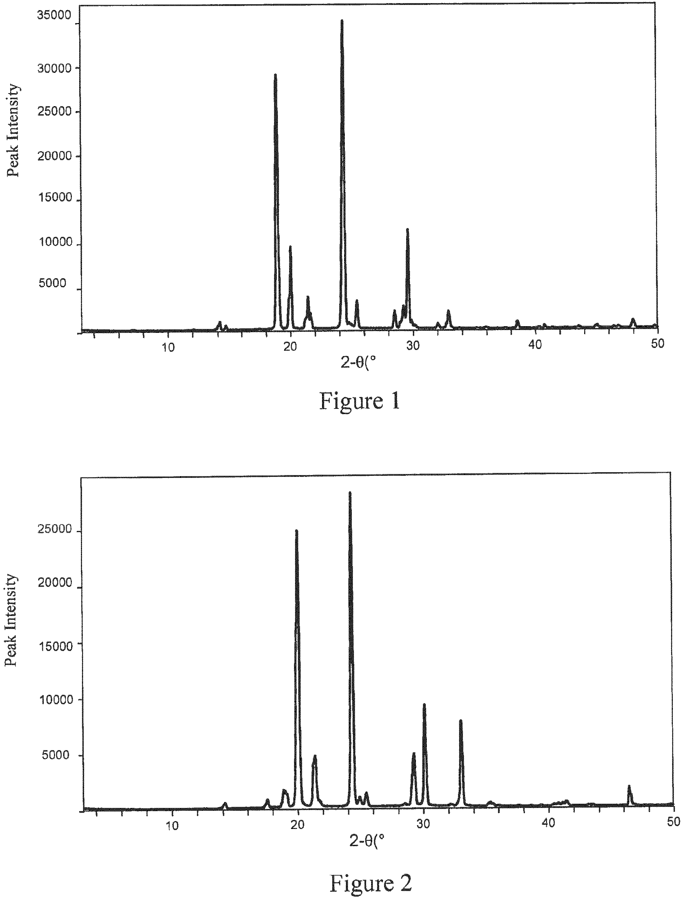

To a mixture of 15 mL of toluene and 6 mL of pyridine 1.6 g of 4-aminopyridine was added. The resulting mixture was heated to 95 °C to obtain a colorless clear solution. Then the resulting solution was stood and cooled to room temperature, kept for 3 h, and filtered under suction. The cake was dried in air to obtain the polymorph I of which the melting point was 151.8-152.2 °C and the X-ray powder diffraction pattern was shown in Fig.1.

### Example 2: Preparation of Polymorph II

Method a: To a mixture of 2 mL of acetone and 2 mL of water 3g of 4-aminopyridine was added. The resulting mixture was heated to 80 °C to obtain a clear solution. Then the resulting solution was stood and cooled to room temperature, kept for 2 h, and filtered under suction. The cake was dried in air to obtain the polymorph II. Melting point: 160-160.8 °C.

Method b: To a mixture of 3 mL of ethanol and 3 mL of water 4.5 g of 4-aminopyridine was added. The resulting mixture was heated to 85 °C to obtain a colorless clear solution. Then the resulting solution was stood and cooled to 5 °C, kept for 3 h, and filtered under suction. The cake was dried in air to obtain the polymorph II.

The X-ray powder diffraction pattern of the polymorph II was shown in Fig.2.

### Example 3: Preparation of Polymorph III

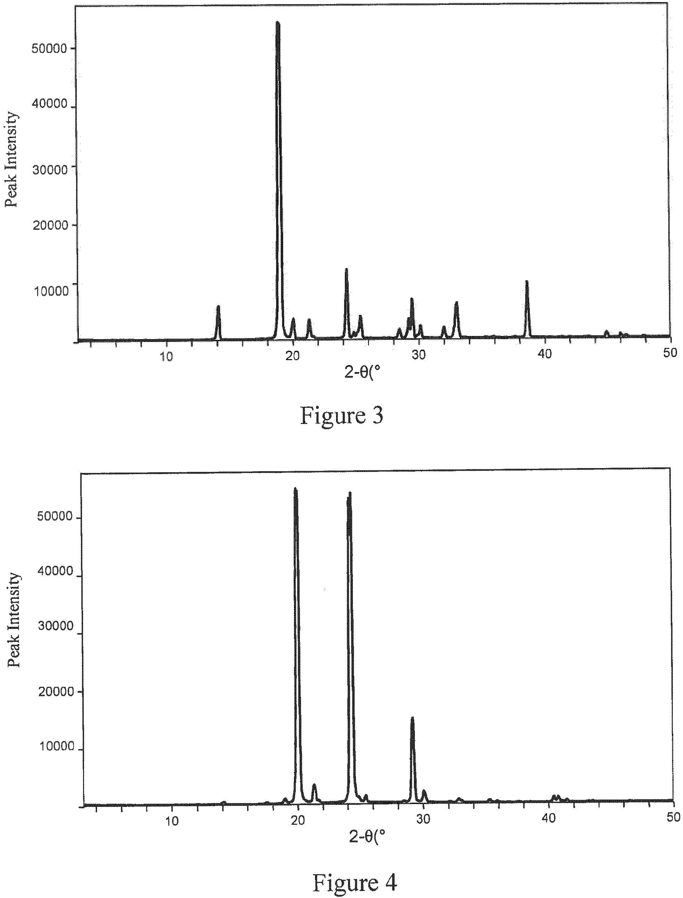

To a mixture of 20 mL of carbon tetrachloride and 5 mL of isopropanol 2.2 g of 4-aminopyridine was added. The resulting mixture was heated to 86 °C and refluxed to obtain a clear solution. Then the resulting solution was stood and cooled to 5 °C, kept for 20 min, and filtered under suction. The cake was dried in air to obtain the polymorph III of which the melting point was 159.8-160.2 °C and the X-ray powder diffraction pattern was shown in Fig.3.

### Example 4: Preparation of Polymorph IV

Method a: 2.8 g of 4-aminopyridine was added to 20 mL of acetone. The resulting mixture was heated to 70 °C to obtain a clear solution. Then the resulting solution was stood and cooled to room temperature, kept for 3 h, and filtered under suction. The cake was dried in air to obtain the polymorph IV. Melting point: 159.8-161.5°C.

Method b: 2 g of 4-aminopyridine was added to 15 mL of tetrahydrofuran. The resulting mixture was heated to 85 °C to obtain a clear solution. Then the resulting solution was stood and cooled to room temperature, kept for 3 h, and filtered under suction. The cake was dried in air to obtain the polymorph IV. The X-ray powder diffraction pattern thereof was shown in Fig.4.

The X-ray powder diffraction pattern of the polymorph IV was shown in Fig.4.

### Example 5: Preparation of Polymorph V

Method a: To a mixture of 2 mL of acetonitrile and 2 mL of water 3 g of 4-aminopyridine was added. The resulting mixture was heated to 80 °C to obtain a clear solution. Then the resulting solution was stood and cooled to 5 °C, kept for 20 min. Flake solids were precipitated. Then the resulting mixture was stood at room temperature for 2 h, and filtered under suction. The cake was dried in air to obtain the polymorph V. Melting point: 159.8-161.5 °C.

Method b: 1.8 g of 4-aminopyridine was added to 20 mL of water. The resulting mixture was heated to 90 °C to obtain a clear solution. Then the resulting solution was stood and cooled to 5 °C, kept for 20 min. Solids were precipitated. Then the resulting mixture was stood at room temperature overnight, and filtered under suction. The cake was dried in air to obtain the polymorph V.

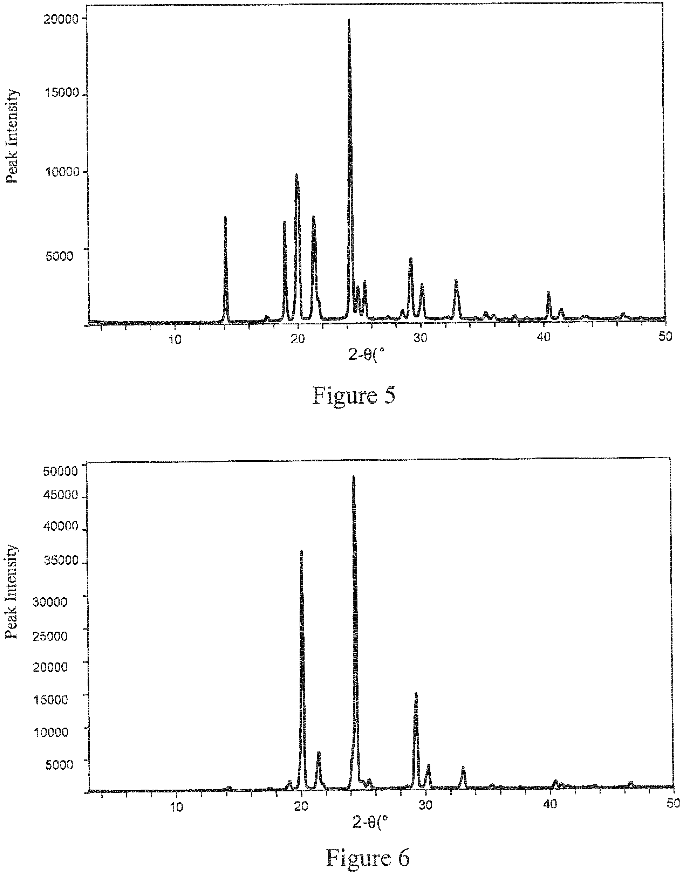

The X-ray powder diffraction pattern of the polymorph V was shown in Fig.5.

### Example 6: Preparation of Polymorph VI

Method a: 2.2 g of 4-aminopyridine was dissolved in a mixture of 16 mL of ethylene glycol dimethyl ether and 4 mL of ethylene glycol monomethyl ether. The resulting mixture was heated to reflux to obtain a clear solution. The resulting solution was stood and cooled to room temperature, and placed in a refrigerator overnight. Solids were precipitated, and filtered under suction. The cake was dried in air to obtain the polymorph VI.

Method b: 1.8 g of 4-aminopyridine was dissolved in 30 mL of ethyl acetate. The resulting mixture was heated to 85 °C to obtain a clear solution. Then the resulting solution was stood and cooled to room temperature, and filtered under suction. The cake was dried in air to obtain the polymorph VI.

Method c: 3 g of 4-aminopyridine was dissolved in 15 mL of acetonitrile. The resulting mixture was heated to 85 °C and refluxed to obtain a clear solution. Then the resulting solution was cooled to room temperature under stirring, and filtered under suction. The cake was dried in air to obtain the polymorph VI.

The X-ray powder diffraction pattern of the polymorph VI was shown in Fig.6.

### Example 7: Preparation of Polymorph VII

Method a: 1.8 g of 4-aminopyridine was dissolved in a mixture of 16 mL of n-hexane and 2.5 mL of N,N-dimethylacetamide. The resulting mixture was heated to 85 °C to obtain a clear solution. The resulting solution was separated into layers and then cooled under stirring, and meanwhile 20 mL of dichloromethane was added dropwise thereto, and the resulting mixture became one phase soon. After the addition of dichloromethane, fine needle crystals were precipitated. The resulting mixture was stood at room temperature for 1h, and filtered under suction. The cake was dried in air to obtain the polymorph VII. Melting point: 160-162 °C.

Method b: 2.8 g of 4-aminopyridine was dissolved in 10 mL of acetonitrile. The resulting mixture was heated to 85 °C to obtain a clear solution. Then the resulting solution was stood and cooled to room temperature, kept for 3 h, and filtered under suction. The cake was dried in air to obtain the polymorph VII.

Method c: 1.8 g of 4-aminopyridine was dissolved in a mixture of 30 mL of chloroform and 1 mL of methanol. The resulting mixture was heated to 75 °C to obtain a clear solution. Then the resulting solution was stood and cooled to room temperature, kept for 3 h, and filtered under suction. The cake was dried in air to obtain the polymorph VII.

Method d: 2.8 g of 4-aminopyridine was dissolved in 20 mL of acetonitrile. The resulting mixture was heated to 70 °C to obtain a clear solution. Then the resulting solution was stood and cooled to room temperature, kept for 3 h, and filtered under suction. The cake was dried in air to obtain the polymorph VII.

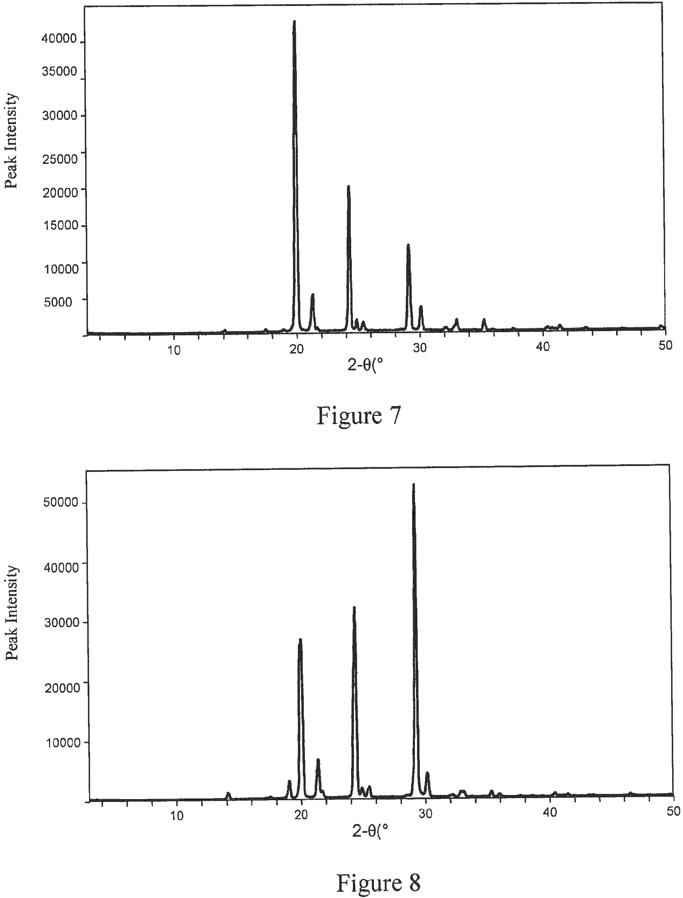

The X-ray powder diffraction pattern of the polymorph VII was shown in Fig.7.

### Example 8: Preparation of Polymorph VIII

3 g of 4-aminopyridine was dissolved in 8 mL of ethanol. The resulting mixture was heated to 85 °C and refluxed for 10 min, then stood and cooled to room temperature, and filtered under suction. The cake was dried in air to obtain the polymorph VIII. Melting point: 157.6-158.5 °C. The X-ray powder diffraction pattern of the polymorph VIII was shown in Fig. 8.

According to Figs. 1-8, the peaks with peak intensity equal or greater than 15 in X-ray powder diffraction patterns of the above-mentioned eight polymorphs of 4-aminopyridine or of the solvates thereof have the following features, respectively.
1. Polymorph I of 4-aminopyridine or of the solvates thereof:

| Diffraction Angle (2θ, °) | Intensity (I/I₀) | Diffraction Angle (2θ, °) | Intensity (I/I₀) |
|---|---|---|---|
| 18.940 | 83.0 | 20.040 | 26.7 |
| 24.381 | 100.0 | 29.601 | 32.1 |

2. Polymorph II of 4-aminopyridine or of the solvates thereof:

| Diffraction angle (2θ, °) | Intensity (I/I₀) | Diffraction angle (2θ, °) | Intensity (I/I₀) |
|---|---|---|---|
| 20.101 | 88.1 | 21.380 | 15.9 |
| 24.361 | 100 | 29.260 | 16.3 |
| 30.140 | 31.9 | 33.039 | 27.1 |

3. Polymorph III of 4-aminopyridine or of the solvates thereof:

| Diffraction angle (2θ, °) | Intensity (I/I₀) | Diffraction angle (2θ, °) | Intensity (I/I₀) |
|---|---|---|---|
| 18.980 | 100.0 | 24.321 | 21.7 |
| 38.619 | 17.5 | | |

4. Polymorph IV of 4-aminopyridine or of the solvates thereof:

| Diffraction angle (2θ, °) | Intensity (I/I₀) | Diffraction angle (2θ, °) | Intensity (I/I₀) |
|---|---|---|---|
| 20.020 | 100.0 | 20.119 | 99.3 |
| 24.259 | 93.4 | 24.419 | 98.5 |
| 29.200 | 26.7 | | |

5. Polymorph V of 4-aminopyridine or of the solvates thereof:

| Diffraction angle (2θ, °) | Intensity (I/I₀) | Diffraction angle (2θ, °) | Intensity (I/I₀) |
|---|---|---|---|
| 14.160 | 35.4 | 18.979 | 33.0 |
| 19.980 | 48.8 | 21.340 | 34.5 |
| 24.361 | 100.0 | 29.259 | 19.6 |

6. Polymorph VI of 4-aminopyridine or of the solvates thereof:

| Diffraction angle (2θ, °) | Intensity (I/I₀) | Diffraction angle (2θ, °) | Intensity (I/I₀) |
|---|---|---|---|
| 20.179 | 76.5 | 24.440 | 100.0 |
| 29.300 | 30.0 | | |

7. Polymorph VII of 4-aminopyridine or of the solvates thereof:

| Diffraction angle (2θ, °) | Intensity (I/I₀) | Diffraction angle (2θ, °) | Intensity (I/I₀) |
|---|---|---|---|
| 20.019 | 100.0 | 24.319 | 46.6 |
| 29.120 | 27.4 | | |

8. Polymorph VIII of 4-aminopyridine or of the solvates thereof:

| Diffraction angle (2θ, °) | Intensity (I/I₀) | Diffraction angle (2θ, °) | Intensity (I/I₀) |
|---|---|---|---|
| 20.060 | 50.5 | 24.401 | 60.6 |
| 29.280 | 100.0 | | |

The above-mentioned eight polymorphs of 4-aminopyridine or of the solvates thereof according to the present invention were exposed to the air at room temperature for 12 months, and then purities thereof were measured by using high performance liquid chromatography. The obtained purity results thereof were shown as follows.

| Polymorph | Purity (%) | Polymorph | Purity (%) |
|---|---|---|---|
| Polymorph I | 99.66 | Polymorph V | 100 |
| Polymorph II | 100 | Polymorph VI | 99.98 |
| Polymorph III | 100 | Polymorph VII | 100 |
| Polymorph IV | 100 | Polymorph VIII | 100 |

The purities were measured by using high performance liquid chromatography under the following conditions.
Chromatographic Column: Agilent extend-C18 (250*4.6 mm, 5 µ)
Mobile Phase: 0.01 mol/L of monopotassium phosphate solution containing 0.01 mol/L of sodium octanesulfonate (adjusted to pH 2.6 with phosphoric acid)-methanol (75:25);
Column Temperature: 40 °C;
Detection Wavelength: 264 nm;
Flow Rate: 1 ml/min.

It can be seen from the above data that the above-mentioned eight polymorphs of 4-aminopyridine or of the solvates thereof according to the present invention still have extremely high purities after exposed to the air at room temperature for 12 months, and no significant degradation product was produced, which suggests that the polymorphs according to the present invention have highly excellent stability.

## Claims

1. A polymorph of 4-aminopyridine or a polymorph of a solvate thereof.

2. The polymorph of 4-aminopyridine or the polymorph of the solvate thereof of claim 1, **characterized in that** X-ray powder diffraction pattern of polymorph I of 4-aminopyridine or of the solvate thereof has the following peaks:
| Diffraction angle (2θ, °) | Intensity (I/I₀) | Diffraction angle (2θ, °) | Intensity (I/I₀) |
|---|---|---|---|
| 18.940 | 83.0 | 20.040 | 26.7 |
| 24.381 | 100.0 | 29.601 | 32.1 |

3. The polymorph of 4-aminopyridine or the polymorph of the solvate thereof of claim 1, **characterized in that** X-ray powder diffraction pattern of polymorph II of 4-aminopyridine or of the solvate thereof has the following peaks:
| Diffraction angle (2θ, °) | Intensity (I/I₀) | Diffraction angle (2θ, °) | Intensity (I/I₀) |
|---|---|---|---|
| 20.101 | 88.1 | 21.380 | 15.9 |
| 24.361 | 100 | 29.260 | 16.3 |
| 30.140 | 31.9 | 33.039 | 27.1 |

4. The polymorph of 4-aminopyridine or the polymorph of the solvate thereof of claim 1, **characterized in that** X-ray powder diffraction pattern of polymorph III of 4-aminonyridine or of the solvate thereof has the following peaks:
| Diffraction angle (2θ, °) | Intensity (I/I₀) | Diffraction angle (2θ, °) | Intensity (I/I₀) |
|---|---|---|---|
| 18.980 | 100.0 | 24.321 | 21.7 |
| 38.619 | 17.5 | | |

5. The polymorph of 4-aminopyridine or the polymorph of the solvate thereof of claim 1, **characterized in that** X-ray powder diffraction pattern of polymorph IV of 4-aminopyridine or of the solvate thereof has the following peaks:
| Diffraction angle (2θ, °) | Intensity (I/I₀) | Diffraction angle (2θ, °) | Intensity (I/I₀) |
|---|---|---|---|
| 20.020 | 100.0 | 20.119 | 99.3 |
| 24.259 | 93.4 | 24.419 | 98.5 |
| 29.200 | 26.7 | | |

6. The polymorph of 4-aminopyridine or the polymorph of the solvate thereof of claim 1, **characterized in that** X-ray powder diffraction pattern of polymorph V of 4-aminopyridine or of the solvate thereof has the following peaks:
| Diffraction angle (2θ, °) | Intensity (I/I₀) | Diffraction angle (2θ, °) | Intensity (I/I₀) |
|---|---|---|---|
| 14.160 | 35.4 | 18.979 | 33.0 |
| 19.980 | 48.8 | 21.340 | 34.5 |
| 24.361 | 100.0 | 29.259 | 19.6 |

7. The polymorph of 4-aminopyridine or the polymorph of the solvate thereof of claim 1, **characterized in that** X-ray powder diffraction pattern of polymorph VI of 4-aminopyridine or of the solvate thereof has the following peaks:
| Diffraction angle (2θ, °) | Intensity (I/I₀) | Diffraction angle (2θ, °) | Intensity (I/I₀) |
|---|---|---|---|
| 20.179 | 76.5 | 24.440 | 100.0 |
| 29.300 | 30.0 | | |

8. The polymorph of 4-aminopyridine or the polymorph of the solvate thereof of claim 1, **characterized in that** X-ray powder diffraction pattern of polymorph VII of 4-aminopyridine or of the solvate thereof has the following peaks:
| Diffraction angle (2θ, °) | Intensity (I/I₀) | Diffraction angle (2θ, °) | Intensity (I/I₀) |
|---|---|---|---|
| 20.019 | 100.0 | 24.319 | 46.6 |
| 29.120 | 27.4 | | |

9. The polymorph of 4-aminopyridine or the polymorph of the solvate thereof of claim 1, **characterized in that** X-ray powder diffraction pattern of polymorph VIII of 4-aminopyridine or of the solvate thereof has the following peaks:
| Diffraction angle (2θ, °) | Intensity (I/I₀) | Diffraction angle (2θ, °) | Intensity (I/I₀) |
|---|---|---|---|
| 20.060 | 50.5 | 24.401 | 60.6 |
| 29.280 | 100.0 | | |

10. A method for preparing the polymorph of 4-aminopyridine or the polymorph of the solvate thereof of any one of claims 1 to 9, **characterized in that** the method comprises
(a) dissolving 4-aminopyridine in a single solvent or a mixed solvent,
(b) heating the mixture obtained in step (a) to obtain a clear solution,
(c) cooling the clear solution obtain in step (b) with or without adding a poor solvent, and
(d) precipitating a solid.

11. A use of the polymorph of 4-aminopyridine or the polymorph of the solvate thereof of any one of claims 1 to 9 in the manufacture of a medicament for treating multiple sclerosis.

12. A formulation comprising the polymorph of 4-aminopyridine or the polymorph of the solvate thereof of any one of claims 1 to 9, **characterized in that** the formulation is a tablet, a capsule, a powder for injection, a solution, an aerosol, a spray, a cream, a patch, an eye drop, an ear drop or an implant, which can be used via injection, oral, inhalation, transdermal, eye drop, ear drop, rectal, or vaginal administration.
